# FASCICULE DE BREVET EUROPEEN

(11) **EP 0 780 115 B1**
(45) Date de publication et mention de la délivrance du brevet: **07.10.1998**
(21) Numéro de dépôt: 96402561.3
(22) Date de dépôt: 27.11.1996
(51) Int. Cl.: A61K 7/00, B01J 13/22

(54) **Nanoparticules enrobées d'une phase lamellaire à base de tensioactif siliconé et compositions les contenant**
Nanopartikel umhüllt mit einer auf Silikontensid basierten lamellaren Phase sowie ihre Zusammensetzungen
Nanoparticles coated with a lamellar phase based on a silicone surfactant and compositions containing them

(30) Priorité: 21.12.1995 FR 9515293
(43) Date de publication de la demande: 25.06.1997
(73) Titulaire: L'OREAL, 75008 Paris (FR)
(72) Inventeur: Simonnet, Jean-Thierry, 75011 Paris (FR); Richart, Pascal, 75013 Paris (FR)
(74) Mandataire: Tezier Herman, Béatrice

(56) Documents cités:
- EP-A- 0 447 318
- EP-A- 0 511 092
- EP-A- 0 641 557
- US-A- 5 364 633
- PATENT ABSTRACTS OF JAPAN vol. 94, no. 011 & JP 06 322236 A (SANYO CHEM IND LTD), 22 Novembre 1994,

## Description

La présente invention se rapporte à des nanoparticules pourvues d'un enrobage lamellaire constitué d'un tensioactif siliconé, et à leur utilisation dans une composition, notamment dans une composition topique pour le traitement de la peau, des muqueuses, des ongles, du cuir chevelu et/ou des cheveux.

On connait, par EP511092, une composition cosmétique comprenant des microsphères enrobées d'une huile fluorée, d'une huile de silicone fluorée ou d'une gomme de silicone.
Il est connu dans les domaines cosmétique et dermatologique, par exemple par les documents EP-A-557489, EP-A-447318 et WO-A-93/25195, d'appliquer sur la peau des actifs piégés dans des sphérules afin d'améliorer l'efficacité de ces actifs. Ces sphérules sont en particulier des nanoparticules de polymère, dont la petite taille, de l'ordre de 10 à 1000 nm, permet une meilleure pénétration dans les couches supérieures de l'épiderme. Le terme de nanoparticules recouvre d'une part les nanosphères et d'autre part les nanocapsules. On désigne par 〈〈 nanosphères 〉〉 les nanoparticules constituées par une matrice polymérique poreuse sur laquelle l'actif est absorbé et/ou adsorbé, et par 〈〈 nanocapsules 〉〉 les nanoparticules constituées par une membrane polymérique qui encapsule un coeur huileux.
Lorsque les nanoparticules sont incorporées dans des compositions, il se produit fréquemment une libération des actifs dans la composition. Dans le cas particulier des nanocapsules, quand elles sont incorporées dans une composition comportant une phase huileuse, telles que par exemple les émulsions, les actifs encapsulés dans le coeur huileux migrent vers la phase huileuse de la composition. Cette libération prématurée des actifs dans la composition rend inefficace leur encapsulation dans des nanoparticules.
Pour pallier à cet inconvénient, il est connu d'enrober les nanoparticules par une phase lamellaire. Jusqu'à présent, pour obtenir un enrobage ayant une étanchéité suffisante, on utilisait un enrobage lamellaire à base de lécithine, comme décrit dans le document EP-A-447318. Or, la lécithine présente l'inconvénient d'être sensible à l'oxydation, ce qui a pour conséquence un jaunissement des nanoparticules, conférant à la composition les contenant, un aspect peu agréable pour l'utilisateur.
Il subsiste donc le besoin de nanoparticules pourvues d'un enrobage lamellaire ayant une bonne étanchéité et ne présentant pas les inconvénients indiqués ci-dessus.

La demanderesse a trouvé de façon inattendue, un enrobage lamellaire permettant d'atteindre cet objectif.

En effet, la demanderesse a trouvé que l'utilisation d'un tensioactif siliconé permettait d'obtenir des nanoparticules enrobées moins sensibles à l'oxydation et donc plus blanches. En outre, grâce à l'emploi d'un tensioactif siliconé, la fabrication des nanoparticules enrobées est réalisée avec un gain de temps appréciable. En effet, la présence du tensioactif siliconé diminue la formation de mousse lors de leur fabrication.

Aussi, la présente invention a pour objet une nanoparticule ayant une dimension allant de 10 à 1000 nm, formée d'un polymère et enrobée d'un enrobage lamellaire, caractérisée en ce que l'enrobage lamellaire est constitué d'au moins un tensioactif siliconé comportant au moins une chaîne oxyéthylénée et/ou oxypropylénée.

Les nanoparticules selon l'invention ont de préférence une dimension allant de 10 à 600 nm.

Un tensioactif siliconé est un composé siliconé comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5364633 et US-A-5411744. Ces documents décrivent l'utilisation de tensioactifs siliconés pour préparer des vésicules lipidiques. Toutefois, ils n'enseignent pas la possibilité de former, autour de nanoparticules, des phases lamellaires à partir de tels tensioactifs.

De préférence, le tensioactif siliconé utilisé selon la présente invention est un composé de formule (I) : 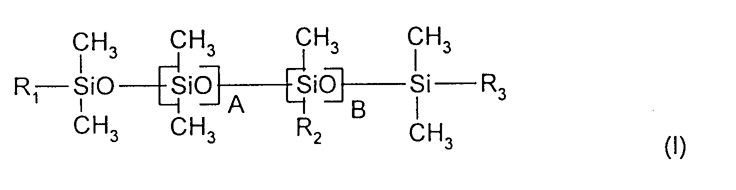 dans laquelle
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) : 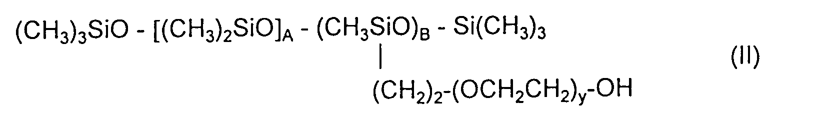 dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (I), les composés de formule (III) :

HO-(OCH₂CH₂)_{y}-(CH₂)₃-[(CH₃)₂SiO]_{A'}-(CH₂)₃-(OCH₂CH₂)_{y}-OH (III)

dans laquelle A' et y sont des nombres entiers allant de 10 à 20.

On peut utiliser comme composés de l'invention ceux vendus par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12.

Le composé Q4-3667 est un composé de formule (III) où A est 15 et y est 13.

Les polymères constitutifs des nanoparticules selon l'invention peuvent être des polymères biodégradables ou non biodégradables. Les nanoparticules en polymères biodégradables pénètrent dans la peau et se dégradent dans l'épiderme sous l'action des enzymes qui y sont présentes, tandis que les nanoparticules en polymères non biodégradables ne pénètrent que dans les couches superficielles du *stratum corneum* et sont éliminées naturellement lors du renouvellement de la peau.

Comme polymères biodégradables, on peut utiliser tout polymère susceptible d'être dégradé par les enzymes de la peau, et notamment ceux cités dans le document EP-A-447318. On peut citer en particulier comme polymères biodégradables les poly-L- et DL-lactides et les polycaprolactones.

Les polymères non-biodégradables utilisables selon l'invention peuvent être choisis parmi tous les polymères qui ne sont pas dégradés par les enzymes de la peau, et notamment ceux cités dans le document EP-A-557489. Parmi les polymères non-biodégradables, on peut citer en particulier les copolymères de chlorure de vinyle et d'acétate de vinyle, et les copolymères d'acide méthacrylique et d'ester méthylique d'acide méthacrylique.

De préférence, les nanoparticules de l'invention sont des nanocapsules. A cet efffet, les polymères décrits ci-dessus sont particulièrement appropriés pour l'obtention de ces nanocapsules.

Dans les nanoparticules de l'invention, le rapport pondéral entre le tensioactif siliconé et le polymère va de manière avantageuse de 0,1 à 2, et de préférence de 0,5 à 1.

Les nanoparticules contiennent généralement au moins un actif piégé, seul ou dans un support. Le rapport pondéral entre le polymère et l'actif et/ou le support va de manière avantageuse de 0,01 à 1, et de préférence de 0,05 à 0,5.

Les nanoparticules selon l'invention sont préparées selon les procédés habituels, et notamment selon les procédés décrits dans les documents EP-A-447318 et WO-A-93/25195.

Selon certains de ces procédés, un agent tensioactif est utilisé au cours de la fabrication des nanoparticules. Comme agent tensioactif utilisable dans ces procédés, on peut citer par exemple les condensats d'oxyde d'éthylène et d'oxyde de propylène, et notamment les poloxamers tels que celui vendu sous la dénomination 〈〈 Pluronic F-68 〉〉 par la société BASF.

Les nanoparticules enrobées selon l'invention sont notamment utilisées dans des compositions à application topique, en particulier des compositions cosmétiques et/ou dermatologiques.

La présente invention a aussi pour objet une composition, plus spécialement à application topique, caractérisée en ce qu'elle contient, dans un milieu approprié, des nanoparticules telles que définies ci-dessus.

Les nanoparticules sont introduites généralement sous forme d'une suspension dans la composition.

Par milieu approprié, on entend le milieu servant à la fabrication des nanoparticules et/ou un milieu topiquement acceptable, c'est-à-dire compatible avec la peau, les muqueuses, les ongles, le cuir chevelu et les cheveux.

De préférence, les nanoparticules représentent de 0,1 à 40 % en poids, et mieux de 5 à 25 % en poids par rapport au poids total de la composition.

Dans la composition selon l'invention, l'actif éventuellement piégé peut être tout actif susceptible d'avoir une activité cosmétique et/ou thérapeutique. Cet actif peut être oléophile ou hydrophile. Il peut être, entre autres, choisi parmi les émollients, les humectants, les agents antiradicalaires, les agents anti-oxydants, les agents anti-inflammatoires, les vitamines, les agents dépigmentants, les agents anti-acnéiques, les agents antiséborrhéiques, les agents kératolytiques, les amincissants, les agents de coloration de la peau, les filtres solaires, les huiles essentielles, les pigments, les agents accélérateurs de bronzage, les parfums, les colorants, les agents mélanorégulateurs, les agents anti-rides et anti-vieillissement, les agents liporégulateurs, les agents antibactériens, les agents antifongiques, les antiperspirants, les déodorants, les agents immunomodulateurs, les agents cicatrisants, les agents protecteurs vasculaires, les agents conditionneurs de la peau et leurs mélanges.

Dans les nanocapsules, l'actif est de préférence un actif oléophile, seul ou en solution dans un support huileux, mais il peut également être sous forme de dispersion, de suspension ou d'émulsion. Le support huileux est de préférence choisi parmi les triglycérides simples ou modifiés, les huiles siliconées, notamment les huiles de silicone volatiles, et les huiles non siliconées, notamment les huiles minérales. On peut citer par exemple comme support huileux les triglycérides d'acide caprylique/ caprique.

Comme actifs piégés utilisables notamment dans les nanocapsules selon l'invention, on peut citer notamment les actifs habituellement utilisés dans les domaines considérés, et par exemple les vitamines telles que le tocophérol (vitamine E) et ses dérivés comme l'acétate de tocophérol, le rétinol (vitamine A) et ses dérivés comme le palmitate de rétinol, les acides gras essentiels et plus particulièrement la vitamine F ; les agents kératolytiques tels que l'acide salicylique et ses dérivés comme notamment ceux décrits dans les documents FR-A-2581542, EP-A-378936 et EP-A-570230, en particulier les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique.

La composition selon l'invention peut se présenter sous toutes les formes appropriées pour une application topique, et notamment sous forme de solution, d'émulsion eau-dans-huile ou huile-dans-eau ou de dispersion aqueuse à base de vésicules lipidiques ioniques et/ou non-ioniques, appelées généralement liposomes. Elle peut constituer par exemple un sérum, un lait ou une crème.

La composition selon l'invention peut aussi se présenter sous forme d'une émulsion huile-dans-eau, les gouttellettes d'huile dispersées dans l'eau étant pourvus d'un enrobage lamellaire, comme décrit dans le document EP-A-641557.

Les quantités des différents constituants des compositions selon l'invention sont celles classiquement utilisées dans le domaine considéré.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans les domaines considérés. L'émulsionnant et le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

De façon connue, la composition de l'invention peut contenir également des adjuvants habituels dans les domaines cosmétique et/ou dermatologique, tels que les gélifiants, les actifs, les conservateurs, les antioxydants, les agents complexants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme huiles utilisables dans l'invention, on peut citer les huiles minérales (huile de vaseline), les huiles végétales (huile d'avocat, huile d'amande douce), les huiles animales, les huiles de synthèse (stéarylheptanoate, triglycérides d'acide caprique/caprylique), les huiles siliconées (huile de silicone volatile, polydiméthylsiloxane), et les huiles fluorées. On peut aussi utiliser comme matières grasses des alcools gras (alcool cétylique), des acides gras (acide stéarique), des cires.

Comme émulsionnants utilisables dans l'invention, on peut citer par exemple le monostéarate de glycérol, le monostéarate de sorbitane oxyéthyléné, le distéarate de sucrose, le polyéthylène glycol éther d'alcool cétéarylique.

Comme gélifiants hydrophiles, on peut citer les polymères carboxyvinyliques (carbomer), les copolymères acryliques, les polyacrylamides, les polysaccharides, les gommes naturelles et les argiles, et, comme gélifiants lipophiles, on peut citer les argiles modifiées comme les bentones, les sels métalliques d'acides gras, l'éthylcellulose ou encore le polyéthylène.

Comme actifs hydrophiles, on peut utiliser les polyols (glycérine), les acides aminés (hydroxyproline), les vitamines hydrosolubles (D-panthénol), les polysaccharides (hyaluronate de sodium).

Comme actifs lipophiles, on peut utiliser le rétinol (vitamine A) et ses dérivés (palmitate de rétinol), le tocophérol (vitamine E) et ses dérivés.

La composition selon l'invention peut notamment être utilisée pour le traitement de la peau, des muqueuses, des ongles, du cuir chevelu et/ou des cheveux, en particulier pour le traitement du vieillissement de la peau (rides, éclat du teint). Aussi, l'invention a encore pour objet l'utilisation de la composition définie ci-dessus pour le traitement cosmétique et/ou dermatologique de la peau contre le vieillissement.

Elle a aussi pour objet un procédé de traitement thérapeutique et/ou non-thérapeutique de la peau et/ou des muqueuses et/ou des ongles et/ou du cuir chevelu et/ou des cheveux, caractérisé en ce qu'il consiste à appliquer sur la peau et/ou les muqueuses et/ou les ongles et/ou le cuir chevelu et/ou les cheveux, la composition telle que définie ci-dessus.

Les exemples ci-après de compositions selon l'invention, sont donnés à titre d'illustration et sans caractère limitatif. Les quantités y sont données en % en poids.

### Exemple 1 : Préparation de nanocapsules enrobées selon l'invention, contenant 5 % d'acétate de tocophérol

Dans un bécher de 500 ml, on dissout 1 g de polycaprolactone, 5 g d'acétate de tocophérol et 1 g de DC 2-5695 dans 200 ml d'acétone sous agitation légère. Dans un autre bécher, on dissout 0,5 g de Pluronic F-68 dans 200 g d'eau distillée sous agitation. On verse ensuite la phase acétonique dans la phase aqueuse en maintenant l'agitation. Puis, on évapore l'acétone et une partie de l'eau avec un évaporateur rotatif jusqu'à obtention de 100 ml de solution. On obtient une suspension de nanocapsules ayant un diamètre moyen de 300 nm.

### Exemple 2 : Préparation de nanocapsules enrobées selon l'invention, contenant 2,5 % de palmitate de rétinol

On procède comme dans l'exemple 1, en remplaçant l'acétate de tocophérol par 5 g d'un mélange composé à 50/50 de palmitate de rétinol et de triglycérides d'acide caprylique/caprique.

### Contre-exemple : Préparation de nanocapsules enrobées selon l'art antérieur, contenant 5 % d'acétate de tocophérol

On procède comme dans l'exemple 1, en utilisant 5 g d'acétate de tocophérol et en remplaçant le DC 2-5695 par 1 g de lécithine.

On a comparé les nanocapsules obtenues selon l'exemple 1 et celles obtenues selon le contre-exemple. Les résultats sont présentés dans le tableau suivant :

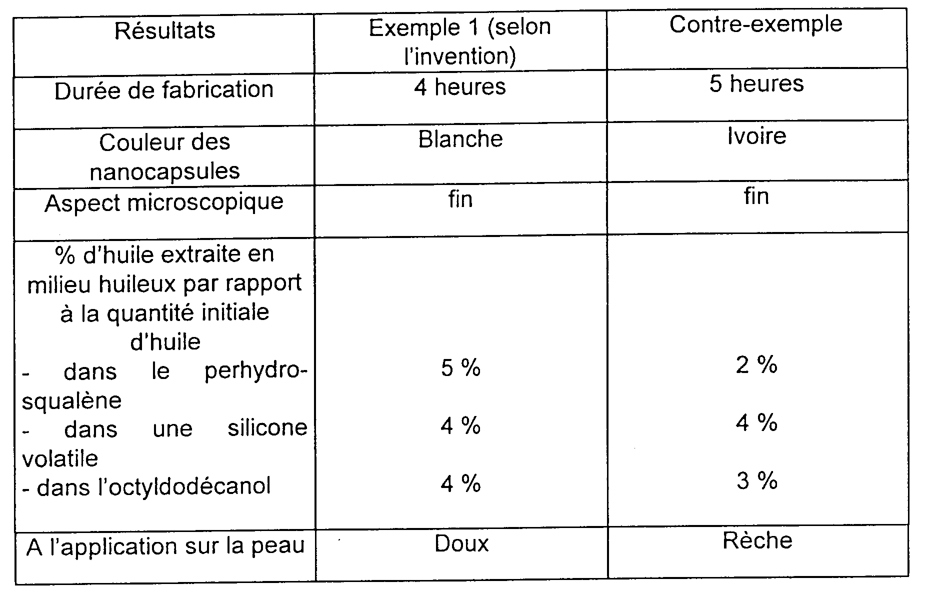

Ces résultats montrent que l'enrobage obtenu avec les tensioactifs siliconés selon l'invention présente une étanchéité aussi bonne que l'enrobage de l'art antérieur tout en ne s'oxydant pas. Par ailleurs, la préparation des nanocapsules enrobées avec les tensioactifs selon l'invention est plus rapide que celle des nanocapsules de l'art antérieur.

### Exemple 3 : Crème de soin de la peau

| | |
|---|---|
| - Nanocapsules obtenues selon l'exemple 1 | 10 % |

| *Phase grasse :* | |
|---|---|
| - Ceteareth-33 (Polyéthylène glycol éther d'alcool cétéarylique) | 0,4 % |
| - Monostéarate de glycérol | 0,8 % |
| - Polydiméthylsiloxane | 0,6 % |
| - Triglycérides d'acide caprique/caprylique | 10 % |
| - Huile de vaseline | 6 % |
| - Alcool cétylique | 0,6 % |

| *Phase aqueuse :* | |
|---|---|
| - Conservateur | 0,3 % |
| - Eau déminéralisée qsp | 100 % |

Le mode opératoire pour préparer cette crème a été le suivant : on a porté séparément la phase grasse et la phase aqueuse à 80°C, puis on a introduit la phase grasse dans la phase aqueuse sous agitation vive et on a maintenu cette agitation jusqu'à ce que la température du mélange atteigne 50°C. On a agité ensuite le mélange plus lentement jusqu'à ce que la température du mélange atteigne 30°C. La suspension de nanocapsules selon l'exemple 1 a été ensuite introduite sous agitation douce.

On a obtenu une crème blanche, apte à améliorer, par application journalière, l'éclat du teint de la peau du visage.

### Exemple 5 : Crème de jour pour le visage

| *Phase A :* | |
|---|---|
| - Distéarate de sucrose | 1,75 % |
| - Stéarate de sorbitan oxyéthyléné (Tween 61 vendu par la société ICI) | 1,15 % |
| - Acide stéarique | 0,75 % |
| - Stéarylheptanoate | 4 % |
| - Huile de vaseline | 1,5 % |
| - Huile d'avocat | 3 % |
| - Huile d'amande douce | 3 % |
| - Huile de silicone volatile | 2,5 % |
| - Palmitate de rétinol à 1500 Ul/mg | 0,5 % |
| - Conservateurs | 0,2 % |

| *Phase B:* | |
|---|---|
| - Glycérine | 5 % |
| - Triéthanolamine | 0,35 % |
| - Eau déminéralisée | 58,3 % |

| *Phase C :* | |
|---|---|
| - Suspension de nanocapsules selon l'exemple 1 | 10 % |

| *Phase D :* | |
|---|---|
| - Carbomer | 0,3 % |
| - Eau déminéralisée | 7,7 % |

La crème obtenue contenant des nanocapsules enrobées comprend également des gouttelettes d'huile pourvues d'un enrobage lamellaire selon le document EP-A-641557.

Le mode opératoire pour préparer cette crème a été le suivant : on a mélangé les constituants de la phase A et on a chauffé le mélange à 60°C ; on a fait de même pour la phase B ; puis on a introduit la phase B dans la phase A sous agitation ; ensuite, on a homogénéisé le mélange obtenu trois fois de suite dans un homogénéisateur haute pression à 500 bars (5.10⁷ Pa). Puis on a ramené le mélange à la température ambiante et on y a introduit la phase C sous faible agitation. On a introduit ensuite délicatement la phase D et on a mélangé doucement jusqu'à homogénéité.

On a obtenu une crème blanche, lisse, brillante et à la texture très fine, pouvant être appliquée quotidiennement, apte à traiter les rides et à améliorer l'éclat du teint.

### Exemple 6: Sérum anti-âge (composition avec liposomes)

| *Phase A :* | |
|---|---|
| - Palmitate de sorbitan (Span 40 vendu par la société ICI) | 0,475 % |
| - Cholestérol | 0,475 % |
| - Sel monosodique de l'acide N-stéaroylglutamique (Acylglutamate HS11 vendu par la société Ajinomoto) | 0,05 % |

| *Phase B :* | |
|---|---|
| - Eau déminéralisée | 15 % |
| - Glycérine | 3 % |
| - Hydroxyproline | 1 % |

| *Phase C :* | |
|---|---|
| - Eau déminéralisée | 52,9 % |
| - Conservateurs | 0,3 % |
| - D-panthénol | 1,5 % |
| - Hyaluronate de sodium | 0,1 % |

| *Phase D :* | |
|---|---|
| - Suspension de nanocapsules de l'exemple 2 | 15 % |

| *Phase E :* | |
|---|---|
| - Carbomer | 0,2 % |
| - Triéthanolamine | 0,2 % |
| - Eau déminéralisée | 9,8 % |

Le mode opératoire pour préparer cette crème a été le suivant : On a introduit la phase A dans la phase B préalablement chauffée à 80°C, sous agitation pendant une heure. Puis on a homogénéisé le mélange trois fois de suite à l'homogénéisateur haute pression à 600 bars (6.10⁷ Pa). On a ensuite ramené le mélange à la température ambiante et on y a introduit la phase C sous faible agitation. On a introduit ensuite délicatement la phase D et on a mélangé doucement jusqu'à homogénéité. Enfin, on a introduit progressivement la phase E et on a maintenu l'agitation jusqu'à homogénéité complète.

On a obtenu un sérum d'une grande fraîcheur à l'application.

## Revendications

1. Nanoparticule ayant une dimension allant de 10 à 1000 nm, formée d'un polymère et enrobée d'un enrobage lamellaire, caractérisée en ce que l'enrobage lamellaire est constitué d'au moins un tensioactif siliconé comportant au moins une chaîne oxyéthylénée et/ou oxypropylénée.

2. Nanoparticule selon la revendication 1, caractérisée en ce que le tensioactif siliconé est un tensioactif siliconé de formule (I) : 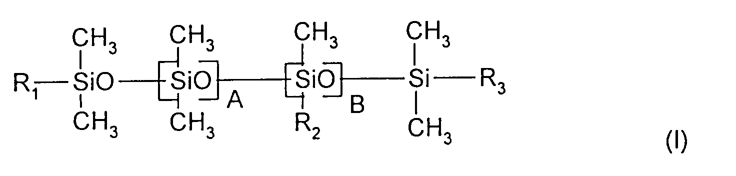 dans laquelle :
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

3. Nanoparticule selon la revendication 2, caractérisée en ce que le tensioactif siliconé est un composé de formule (I) où le radical alkyle est un radical méthyle, x est un nombre entier allant de 2 à 6 et y est un nombre entier allant de 4 à 30.

4. Nanoparticule selon l'une quelconque des revendications précédentes, caractérisée en ce que le tensioactif siliconé est un composé de formule (II) : 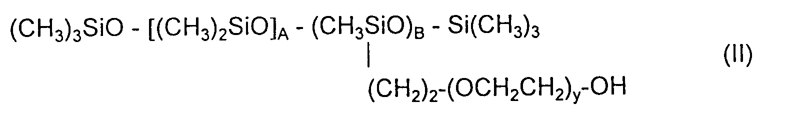 dans laquelle A est un nombre entier allant de 20 à 105, B est un nombre entier allant de 2 à 10 et y est un nombre entier allant de 10 à 20.

5. Nanoparticule selon la revendication précédente, caractérisée en ce que le tensioactif siliconé est choisi parmi les composés de formule (II) où A est 22, B est 2 et y est 12 ; A est 103, B est 10 et y est 12 ; A est 27, B est 3 et y est 12.

6. Nanoparticule selon l'une quelconque des revendications précédentes, caractérisée en ce que le tensioactif siliconé est un composé de formule (III) :
HO-(OCH₂CH₂)_{y}-(CH₂)₃-[(CH₃)₂SiO]_{A'}-(CH₂)₃-(OCH₂CH₂)_{y}-OH (III)
dans laquelle A' et y sont des nombres entiers allant de 10 à 20.

7. Nanoparticule selon la revendication précédente, caractérisée en ce que le tensioactif siliconé est un composé de formule (III) où A est 15 et y est 13.

8. Nanoparticule selon l'une quelconque des revendications précédentes, caractérisée en ce qu'elle a une dimension allant de 10 à 600 nm.

9. Nanoparticule selon l'une quelconque des revendications précédentes,, caractérisée en ce que le polymère est choisi dans le groupe comprenant les poly-L- et DL-lactides, les polycaprolactones, les copolymères de chlorure de vinyle et d'acétate de vinyle et les copolymères d'acide méthacrylique et d'ester méthylique d'acide méthacrylique.

10. Nanoparticule selon l'une quelconque des revendications précédentes, caractérisée en ce que le polymère est un polymère biodégradable.

11. Nanoparticule selon l'une quelconque des revendications précédentes, caractérisée en ce qu'elle est une nanocapsule.

12. Nanoparticule selon l'une quelconque des revendications précédentes, caractérisée en ce que le rapport pondéral entre le tensioactif siliconé et le polymère va de de 0,1 à 2.

13. Nanoparticule selon l'une quelconque des revendications précédentes, caractérisée en ce qu'elle contient au moins un actif.

14. Nanoparticule selon la revendication précédente, caractérisée en ce que le rapport pondéral entre le polymère et l'actif va de 0,01 à 1.

15. Nanoparticule selon la revendication 13 ou 14, caractérisée en ce que l'actif est un actif oléophile.

16. Nanoparticule selon l'une quelconque des revendications 13 à 15, caractérisée en ce que l'actif est choisi dans le groupe comprenant le rétinol (vitamine A) et ses dérivés, le tocophérol (vitamine E) et ses dérivés.

17. Composition à application topique, caractérisée en ce qu'elle contient, dans un milieu approprié, des nanoparticules selon l'une quelconque des revendications 1 à 16.

18. Composition selon la revendication précédente, caractérisée en ce que les nanoparticules représentent de 0,1 à 40 % en poids du poids total de la composition.

19. Composition selon la revendication 17 ou 18, caractérisée en ce qu'elle se présente sous forme d'une émulsion ou d'une dispersion de vésicules lipidiques.

20. Composition selon l'une quelconque des revendications 17 à 19, caractérisée en ce qu'elle constitue une composition cosmétique et/ou dermatologique.

21. Utilisation de la composition selon l'une quelconque des revendications 17 à 20 pour le traitement cosmétique de la peau contre le vieillissement.

22. Utilisation de nanoparticules selon l'une quelconque des revendications 1 à 16 pour la préparation d'une composition dermatologique destinée au traitement de la peau contre le vieillissement.

## Claims

1. Nanoparticle ranging in size from 10 to 1000 nm, composed of a polymer and coated with a lamellar coating, characterized in that the lamellar coating is composed of at least one silicone surfactant containing at least one oxyethylenated and/or oxypropylenated chain.

2. Nanoparticle according to Claim 1, characterized in that the silicone surfactant is a silicone surfactant of formula (I): 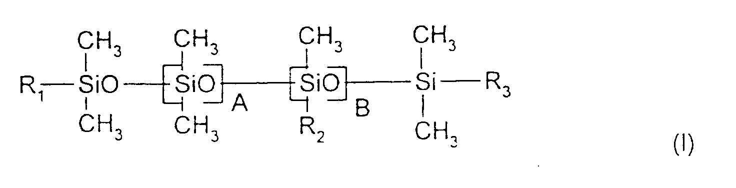 in which:
R₁, R₂ and R₃, independently of one another, represent a C₁-C₆-alkyl radical or a radical (CH₂)ₓ-(OCH₂CH₂)_{y}-(OCH₂CH₂CH₂)_{z}-OR₄, at least one radical R₁,
R₂ or R₃ not being an alkyl radical; R₄ being a hydrogen, an alkyl radical or an acyl radical;
A is an integer ranging from 0 to 200;
B is an integer ranging from 0 to 50; on condition that A and B are not equal to zero at the same time;
x is an integer ranging from 1 to 6;
y is an integer ranging from 1 to 30;
z is an integer ranging from 0 to 5.

3. Nanoparticle according to Claim 2, characterized in that the silicone surfactant is a compound of formula (I) in which the alkyl radical is a methyl radical, x is an integer ranging from 2 to 6 and y is an integer ranging from 4 to 30.

4. Nanoparticle according to any one of the preceding claims, characterized in that the silicone surfactant is a compound of formula (II): 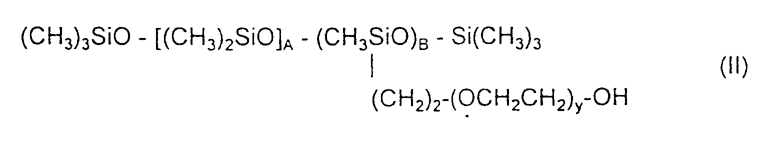 in which A is an integer ranging from 20 to 105, B is an integer ranging from 2 to 10 and y is an integer ranging from 10 to 20.

5. Nanoparticle according to the preceding claim, characterized in that the silicone surfactant is chosen from the compounds of formula (II) in which A is 22, B is 2 and y is 12; A is 103, B is 10 and y is 12; A is 27, B is 3 and y is 12.

6. Nanoparticle according to any one of the preceding claims, characterized in that the silicone surfactant is a compound of formula (III):
HO-(OCH₂CH₂)_{y}-(CH₂)₃-[(CH₃)₂SiO]_{A'}-(CH₂)₃-(OCH₂CH₂)_{y}-OH (III)
in which A' and y are integers ranging from 10 to 20.

7. Nanoparticle according to the preceding claim, characterized in that the silicone surfactant is a compound of formula (III) in which A is 15 and y is 13.

8. Nanoparticle according to any one of the preceding claims, characterized in that it ranges in size from 10 to 600 nm.

9. Nanoparticle according to any one of the preceding claims, characterized in that the polymer is chosen from the group comprising poly-L- and -DL-lactides, polycaprolactones, copolymers of vinyl chloride and vinyl acetate and copolymers of methacrylic acid and methacrylic acid methyl ester.

10. Nanoparticle according to any one of the preceding claims, characterized in that the polymer is a biodegradable polymer.

11. Nanoparticle according to any one of the preceding claims, characterized in that it is a nanocapsule.

12. Nanoparticle according to any one of the preceding claims, characterized in that the weight ratio of the silicone surfactant to the polymer ranges from 0.1 to 2.

13. Nanoparticle according to any one of the preceding claims, characterized in that it contains at least one active agent.

14. Nanoparticle according to the preceding claim, characterized in that the weight ratio of the polymer to the active agent ranges from 0.01 to 1.

15. Nanoparticle according to Claim 13 or 14, characterized in that the active agent is an oleophilic active agent.

16. Nanoparticle according to any one of Claims 13 to 15, characterized in that the active agent is chosen from the group comprising retinol (vitamin A) and its derivatives and tocopherol (vitamin E) and its derivatives.

17. Composition for topical application, characterized in that it contains nanoparticles according to any one of Claims 1 to 16 in a suitable medium.

18. Composition according to the preceding claim, characterized in that the nanoparticles represent from 0.1 to 40 % by weight of the total weight of the composition.

19. Composition according to Claim 17 or 18, characterized in that it takes the form of an emulsion or of a dispersion of lipid vesicles.

20. Composition according to any one of Claims 17 to 19, characterized in that it constitutes a cosmetic and/or dermatological composition.

21. Use of the composition according to any one of Claims 17 to 20 for the cosmetic treatment of the skin against ageing.

22. Use of nanoparticles according to any one of Claims 1 to 16 for the preparation of a dermatological composition intended for the treatment of the skin against ageing.

## Patentansprüche

1. Nanopartikel mit einer Größe von 10 bis 1000 nm, die aus einem Polymer bestehen und mit einer lamellaren Umhüllung umhüllt sind, dadurch gekennzeichnet, daß die lamellare Umhüllung aus mindestens einem siliconhaltigen grenzflächenaktiven Stoff besteht, der mindestens eine ethoxylierte und/oder propoxylierte Kette aufweist.

2. Nanopartikel nach Anspruch 1, dadurch gekennzeichnet, daß der siliconhaltige grenzflachenaktive Stoff ein siliconhaltiger grenzflächenaktiver Stoff der Formel (I) ist: 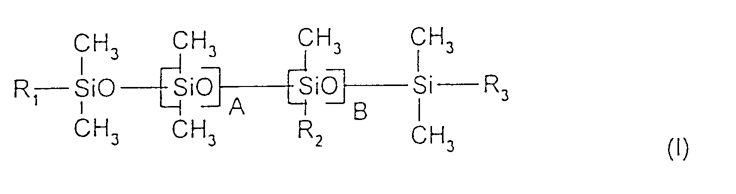 worin bedeuten:
- R₁, R₂ und R₃ unabhängig voneinander eine C₁₋₆-Alkylgruppe oder eine Gruppe -(CH₂)ₓ -(OCH₂CH₂)_{y} -(OCH₂CH₂CH₂)_{z} -OR₄, wobei mindestens eine Gruppe R₁, R₂ oder R₃ keine Alkylgruppe ist und R₄ Wasserstoff, eine Alkylgruppe oder eine Acylgruppe ist,
- A Null oder eine ganze Zahl von 1 bis 200,
- B Null oder eine ganze Zahl von 1 bis 50, mit der Maßgabe, daß A und B nicht gleichzeitig Null bedeuten,
- x eine ganze Zahl von 1 bis 6,
- y eine ganze Zahl von 1 bis 30 und
- z Null oder eine ganze Zahl von 1 bis 5.

3. Nanopartikel nach Anspruch 2, dadurch gekennzeichnet, daß der siliconhaltige grenzflächenaktive Stoff eine Verbindung der Formel (I) ist, worin die Alkylgruppe eine Methylgruppe, x eine ganze Zahl von 2 bis 6 und y eine ganze Zahl von 4 bis 30 bedeuten.

4. Nanopartikel nach einem der vorangehenden Ansprüche, dadurch gekennzeichnet, daß der siliconhaltige grenzflächenaktive Stoff eine Verbindung der Formel (II) ist: 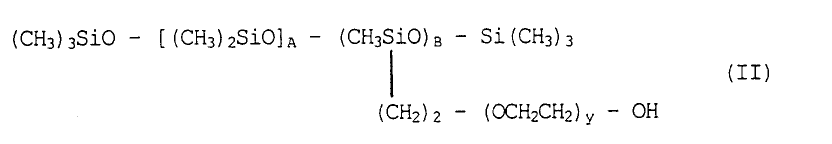 worin bedeuten:
- A eine ganze Zahl von 20 bis 105,
- B eine ganze Zahl von 2 bis 10 und
- y eine ganze Zahl von 10 bis 20.

5. Nanopartikel nach dem vorangehenden Anspruch, dadurch gekennzeichnet, daß der siliconhaltige grenzflächenaktive Stoff unter den Verbindungen der Formel (II), worin A 22, B 2 und y 12; A 103, B 10 und y 12 oder A 27, B 3 und y 12 bedeuten, ausgewählt ist.

6. Nanopartikel nach einem der vorangehenden Ansprüche, dadurch gekennzeichnet, daß der siliconhaltige grenzflächenaktive Stoff eine Verbindung der Formel (III) ist:
HO-(OCH₂CH₂)_{y}-(CH₂)₃-[(CH₃)₂SiO]_{A'}-(CH₂)₃-(OCH₂CH₂)_{y}-OH (III)
worin A' und y ganze Zahlen von 10 bis 20 bedeuten.

7. Nanopartikel nach dem vorangehenden Anspruch, dadurch gekennzeichnet, daß der siliconhaltige grenzflächenaktive Stoff eine Verbindung der Formel (III), worin A 15 und y 13 bedeuten, ist.

8. Nanopartikel nach einem der vorangehenden Ansprüche, dadurch gekennzeichnet, daß sie eine Größe von 10 bis 600 nm aufweisen.

9. Nanopartikel nach einem der vorangehenden Ansprüche, dadurch gekennzeichnet, daß das Polymer unter den Poly-L-lactiden und Poly-DL-lactiden, den Polycaprolactonen, den Copolymeren von Vinylchlorid und Vinylacetat und den Copolymeren von Methacrylsäure und Methacrylsäuremethylester ausgewählt ist.

10. Nanopartikel nach einem der vorangehenden Ansprüche, dadurch gekennzeichnet, daß das Polymer ein biologisch abbaubares Polymer ist.

11. Nanopartikel nach einem der vorangehenden Ansprüche, dadurch gekennzeichnet, daß sie Nanokapseln sind.

12. Nanopartikel nach einem der vorangehenden Ansprüche, dadurch gekennzeichnet, daß das Gewichtsverhältnis des siliconhaltigen grenzflächenaktiven Stoffs zu dem Polymer im Bereich von 0,1 bis 2 liegt.

13. Nanopartikel nach einem der vorangehenden Ansprüche, dadurch gekennzeichnet, daß sie mindestens einen Wirkstoff enthalten.

14. Nanopartikel nach dem vorangehenden Anspruch, dadurch gekennzeichnet, daß das Gewichtsverhältnis des Polymers zu dem Wirkstoff im Bereich von 0,01 bis 1 liegt.

15. Nanopartikel nach Anspruch 13 oder 14, dadurch gekennzeichnet, daß der Wirkstoff ein oleophiler Wirkstoff ist.

16. Nanopartikel nach einem der Ansprüche 13 bis 15, dadurch gekennzeichnet, daß der Wirkstoff unter Retinol (Vitamin A) und seinen Derivaten sowie Tocopherol (Vitamin E) und seinen Derivaten ausgewählt ist.

17. Zusammensetzung zur topischen Anwendung, dadurch gekennzeichnet, daß sie in einem geeigneten Medium Nanopartikel nach einem der Ansprüche 1 bis 16 enthält.

18. Zusammensetzung nach dem vorangehenden Anspruch, dadurch gekennzeichnet, daß die Nanopartikel 0,1 bis 40 Gew.-%, bezogen auf das Gesamtgewicht der Zusammensetzung, ausmachen.

19. Zusammensetzung nach Anspruch 17 oder 18, dadurch gekennzeichnet, daß sie in Form einer Emulsion oder einer Dispersion von Lipidvesikeln vorliegt.

20. Zusammensetzung nach einem der Ansprüche 17 bis 19, dadurch gekennzeichnet, daß sie eine kosmetische und/oder dermatologische Zusammensetzung darstellt.

21. Verwendung der Zusammensetzung nach einem der Ansprüche 17 bis 20 zur kosmetlschen Behandlung der Haut gegen Alterung.

22. Verwendung von Nanopartikeln nach einem der Ansprüche 1 bis 16 zur Herstellung einer dermatologischen Zusammensetzung, die zur Behandlung der Haut gegen Alterung bestimmt ist.
